**Europäisches Patentamt**

**European Patent Office**

**Office européen des brevets**

(19)

(11) Veröffentlichungsnummer: **0 005 856**
B1

(12) EUROPÄISCHE PATENTSCHRIFT

(45) Veröffentlichungstag der Patentschrift: **17.11.82**

(21) Anmeldenummer: **79101745.2**

(22) Anmeldetag: **01.06.79**

(51) Int. Cl.³: **C 07 C 85/08, C 07 C 87/40**

(54) **Verfahren zur Herstellung von Diaminen.**

(30) Priorität: **03.06.78 DE 2824423**

(43) Veröffentlichungstag der Anmeldung:
**12.12.79 Patentblatt 79/25**

(45) Bekanntmachung des Hinweises auf die Patenterteilung:
**17.11.82 Patentblatt 82/46**

(84) Benannte Vertragsstaaten:
**FR GB IT SE**

(56) Entgegenhaltungen:
**DE - A - 2 060 703**
**DE - A - 2 647 317**
**FR - A - 1 525 149**

(73) Patentinhaber: **Ruhrchemie Aktiengesellschaft**
**Bruchstrasse 219**
**D-4200 Oberhausen (DE)**

(72) Erfinder: **Cornils, Boy, Dr. Dipl.-Chem.**
**Friedrich-Ebert-Strasse 45**
**D-4220 Dinslaken (DE)**
Erfinder: **Konkol, Werner, Dr. Dipl.-Chem.**
**Lützowstrasse 40a**
**D-4200 Oberhausen 13 (DE)**
Erfinder: **Diekhaus, Gerhard, Dr. Dipl.-Chem.**
**Walsumermarkstrasse 89**
**D-4200 Oberhausen 14 (DE)**
Erfinder: **Wiebus, Ernst**
**Ferdinandstrasse 77**
**D-4200 Oberhausen 14 (DE)**

Courier Press, Leamington Spa, England.

# 0 005 856

Verfahren zur Herstellung von Diaminen

Die Erfindung betrifft ein zweistufiges Verfahren zur Herstellung von aliphatischen Diaminen, wobei zunächst Dialdehyde mit Monoaminen zu den entsprechenden Diazomethinen umgesetzt und die Diazomethine anschließend in Gegenwart von Ammoniak hydriert werden (aminierende Hydrierung).

Diamine, insbesondere $\alpha$-,$\omega$-Alkylendiamine, z.B. Hexamethylendiamin aber auch cycloaliphatische Diamine, wie 1,4-Diaminocyclohexan sind wertvolle Zwischenprodukte für zahlreiche chemische Synthesen. Sie finden bei der Herstellung von Polyamiden oder von Polyurethanen ebenso Anwendung, wie als Härterkomponente für Epoxidharze.

Aliphatische oder cycloaliphatische Diamine können durch Hydrierung der entsprechenden Dinitrile gewonnen werden. Da die Herstellung der Dinitrile jedoch technisch aufwendig ist, eröffent dieser Weg nur in Sonderfällen einen wirtschaftlichen Zugang zu Diaminen.

Eine andere Arbeitsweise zur Herstellung von Diaminen geht von Dialkoholen aus, die mit Ammoniak umgesetzt werden. Da die Reaktion hohe Temperaturen, die die Bildung von Nebenprodukten begünstigt, erfordert, ist die Ausbeute nur gering.

Statt Dialkohole mit Ammoniak umzusetzen, ist es weiterhin bekannt, Dialdehyde in Gegenwart von Katalysatoren mit Ammoniak und Wasserstoff in einem Schritt zur Reaktion zu bringen. Aber auch bei diesem Verfahren sind die erzielbaren Ausbeuten unbefriedigend, weil die Aldehyde Additions- und Kondensationsreaktionen eingehen und Polyimine gebildet werden.

In diesem Zusammenhang sei auf die DE—OS 26 47 317 hingewiesen, in der die Umsetzung von Dialdehyden zu Diaminen in einem Zweistufenprozess beschrieben wird. Ein wesentliches Merkmal der Umsetzung ist die Einhaltung von Temperaturen unter 25°C und vorzugsweise unter 5°C bei der Herstellung der ersten Stufe (Diimin). Vergleichsbeispiele zeigen, daß höhere Temperaturen zu einer deutlichen Abnahme der Ausbeute führen. Die Anwendung der niedrigen Temperaturen hat aber eine beträchtliche Verminderung der Reaktionsgeschwindigkeit zur Folge, so daß das vorbeschriebene Verfahren für eine wirtschaftliche Gewinnung von Diaminen aus Dialdehyden wenig geeignet ist.

Wegen der großen Bedeutung der Diamine bestand daher die Aufgabe, ein Verfahren zu entwickeln, das die Herstellung dieser Verbindungsklasse in hoher Ausbeute und mit technisch vertretbarem Aufwand gestattet.

Überraschenderweise wurde gefunden, daß man zur Herstellung von aliphatischen oder cycloaliphatischen Diaminen mit 4 bis 18 Kohlenstoffatomen mit Erfolg in der Weise arbeitet, daß man in einer ersten Stufe aliphatische und cycloaliphatische Dialdehyde drucklos bei Temperaturen bis 100° mit Monoaminen zu den entsprechenden Diazomethinen umsetzt und diese in einer zweiten Reaktionsstufe bei 60 bis 200°C und 50 bis 300 bar mit Ammoniak und Wasserstoff in Gegenwart von Hydrierungskatalysatoren unter Abspaltung der Monoamine in die Diamine überführt.

Das erfindungsgemäße Verfahren ermöglicht die Herstellung von Diaminen aus Dialdehyden in Ausbeuten, die 95% übersteigen. In der FR—PS 15 25 149 ist ein Verfahren zur Herstellung primärer Monoamine beschrieben. Hierbei wird eine Carbonylverbindung durch Umsetzung mit einem primären Amin in ein Imin überführt und dieses in Gegenwart von Ammoniak unter Rückgewinnung des primären Amins katalytisch hydriert.

Diese Arbeitsweise stimmt zwar in der Art der Reaktionsdurchführung mit dem erfindungsgemäßen Prozeß überein, unterscheidet sich von diesem aber durch die Einsatzstoffe. Während nach der Vorveröffentlichung monofunktionelle Verbindungen, d.h. Verbindungen, die eine Carbonylgruppe enthalten, eingesetzt werden, geht das neue Verfahren von bifunktionellen Verbindungen aus, nämlich aliphatischen oder cycloaliphatischen Dialdehyden.

Auf Grund der Bifunktionalität sind Dialdehyde erheblich reaktiver als Monocarbonylverbindungen. Dementsprechend ist bei der Umsetzung mit einem primären Amin nicht ohne weiteres die Bildung einer Schiffschen Base zu erwarten, vielmehr ist davon auszugehen, daß sich durch Kondensationsreaktionen irreversibel hochmolekulare Verbindungen bilden. Darüber hinaus ist nicht auszuschließen, daß bereits gebildete Diamine mit noch vorhandenen Dialdehyden ebenfalls polymere Verbindungen ergeben. Im Hinblick auf diese verschiedenartigen Reaktionsmöglichkeiten ist as überraschend, daß der erfindungsgemäße Prozeß selektiv zu Diaminen führt.

Die neue Arbeitsweise ist geeignet zur Herstellung von aliphatischen und cycloaliphatischen Diaminen mit 4 bis 18 Kohlenstoffatomen z.B. von 1,6-Hexamethylen-diamin, 1,8-Octamethylendiamin, 1,12-Dodecamethylendiamin und den isomeren Bis(Aminomethyl)-tricyclo[5.2.1.0$^{2.6}$]-decanen.

Als Ausgangsstoffe gelangen aliphatische oder cycloaliphatische Dialdehyde zum Einsatz. Die Stellung der beiden Carbonylgruppen zueinander im Dialdehydmolekül ist beliebig, bevorzugte aliphatische Dialdehyde sind die $\alpha$, -Verbindungen. Beispiele für Dialdehyde, die nach dem erfindungsgemäßen Verfahren umgesetzt werden können sind 1,4-Butandial, 1,6-Hexandial, 1,2-Dodecandial, Bisformyltricyclo[5.2.1.0$^{2.6}$]-decan. Die Herstellung der Dialdehyde erfolgt nach bekannten Verfahren, beispielsweise aus Diolefinen durch Hydroformylierung, d.h. Umsetzung mit einem Gemisch aus Wasserstoff und Kohlenmonoxid in Gegenwart geeigneter Katalysatoren, wie Rhodium oder Kobalt in feinverteilter Form oder Verbindungen des Rhodiums bzw. Kobalts.

Zur Umsetzung der Dialdehyde in der ersten Reaktionsstufe verwendet man Monoamine, vorzugs-

2

weise solche mit 3 bis 18 Kohlenstoffatomen. Geeignet sind z.B. n-Propylamin, i-Propylamin, n-Butylamin, sek. Butylamin, tert.-Butylamin, die isomeren Pentylamine, Hexylamin sowie 2-Äthylhexylamin, i-Tridecylamin, i-Octadecylamin. Die Umsetzung erfolgt drucklos bei Temperaturen von 10 bis 100°C. Die Reaktionstemperatur wird nach dem Siedeverhalten der Reaktanten gewählt.

Zweckmäßigerweise setzt man das Monoamin in einem Überschuß von 10 bis 100 Mol% ein und fügt den Dialdehyd anteilweise dem Amin zu. Bewährt hat es sich, je Mol Dialdehyd mindestens 2,4 Mole Monoamin zu verwenden. Die Gegenwart eines Lösungsmittels für die Reaktanten und/oder das Reaktionsprodukt ist nicht unbedingt erforderlich, kann in Sonderfällen aber zweckmäßig sein. Dann verwendet man bevorzugt aliphatische oder aromatische Kohlenwasserstoffe wie Toluol, Xylol als Reaktionsmedium.

Das in der ersten Reaktionsstufe gebildete Diazomethin wird ohne Abtrennung von den begleitenden Reaktions-partnern und Nebenprodukten und ohne vorherige Reinigung in Gegenwart von Katalysatoren aminierend hydriert.

Geeignet für die aminierende Hydrierung sind die üblichen Hydrierkatalysatoren. Hierzu zählen Katalysatoren, die ein oder mehrere Elemente der VIII. Gruppe des Periodensystems als Metall und/oder Oxid enthalten. Die aktive Komponente des Katalysators kann allein oder in Kombination mit einem Träger eingesetzt werden. Als Träger kommen z.B. Tonerde, Kieselgur, Kieselsäure und Silikate, insbesondere Aluminiumsilikate in Betracht. Für den Einsatz im erfindungsgemäßen Prozeß geeignete Katalysatoren sind beispielsweise durch Fällung oder Tränkung hergestellte Nickel-, Kobalt-, Rhodium- oder Platinkatalysatoren, wie Nickel oder Kobalt auf Aluminiumoxid mit einem Nickel- oder Kobaltgehalt bis 75 Gew.%. Vorteilhaft enthalten die zuletzt genannten Katalysatoren Magnesium, Mangan und/oder Chrom in Form ihrer Verbindungen als Promotoren.

Die Hydrierung kann mit suspendiertem oder fest angeordnetem Katalysator sowohl in der Flüssig- als such in der Gasphase durchgeführt werden. Sie erfolgt in Gegenwart von mindestens 2 Molen, bevorzugt 4 bis 20 Molen Ammoniak und 1 bis 4 Molen Wasserstoff je Mol Diazomethin. Die Gegenwart eines Lösungsmittels wie Alkohole, Äther oder Kohlenwasserstoffe ist möglich aber nicht zwingend erforderlich. Die Reaktionstemperatur beträgt 60 bis 200°C, der Gesamtdruck 50 bis 300 bar. Zur Aufrechterhaltung des gewünschten Druckes führt man dem Reaktionsgemisch kontinuierlich Wasserstoff zu.

Die Reaktion kann in Reaktoren konventioneller Bauart vorgenommen werden. Zur Durchführung der ersten Reaktionsstufe sind z.B. Rohrreaktoren geeignet, doch können auch Rührkessel verwendet werden. Die aminierende Hydrierung nimmt man in Autoklaven und bevorzugt in Rohrreaktoren vor.

Bei der aminierenden Hydrierung in der zweiten Synthesestufe bildet sich das in der ersten Synthesestufe eingesetzte Monoamin wieder zurück. Es entsteht dabei in einer solchen Menge, daß es den Monoamin-Bedarf der ersten Reaktionsstufe deckt. Bevorzugt führt man daher beide Synthesestufen kontinuierlich durch, unter Rückführung des in der zweiten Reaktionsstufe gebildeten Monoamins in die ersten Reaktionsstufe.

Vergleichsbeispiel

Am Fuß eines senkrecht stehenden Hochdruckrohres mit 2 Volumenteilen Inhalt wird mit einem Durchsatz von 0,27 V/Vh 3 (4), 8 (9)-Bisformyl-tricyclo$[5.2.1.0^{2.6}]$-decan, versetzt mit 1 Gew.% eines feinverteilten Katalysators, der etwa 55% Nickel neben 4 bis 6% Magnesiumoxid als Aktivator und 30 bis 35% kieselsäurehaltiges Trägermaterial enthält, eingepumpt. Gleichzeitig werden je Mol Dialdehyd 10 Mol Ammoniak zugeführt. Bei 140°C und einem durch kontinuierliche Zugabe von Wasserstoff bei 80 bar gehaltenen Gesamtdruck enthält das den Reaktor am Kopf verlassende Reaktionsproduktwasser-, ammoniak- und katalysatorfrei gerechnet—etwa 10% 3(4)-Aminomethyl-tricyclo$[5.2.1.0^{2.6}]$-decan, 35% 3(4),4(9)-Bis-(aminomethyl)-tricyclo$[5.2.1.0^{2.6}]$-decan, 5% 3(4)-Hydroxymethyl-8(9)-aminomethyltricyclo$[5.2.1.0^{2.6}]$-decan und 50% höhersiedende Anteile.

Die Ausbeute an 3(4),8(9)-Bis-(aminomethyl)-tricyclo$[5.2.1.0^{2.6}]$-decan deträgt etwa 44% der Theorie, bezogen auf den eingesetzten Dialdehyd.

Beispiel

In den im Vergleichsbeispiel beschriebenen Reaktor wird mit einem Durchsatz von 0,27 V/Vh ein durch Umsetzung von 3(4),8(9)-Bisformyltricyclo$[5.2.1.0^{2.6}]$-decan und n-Butylamin (100% Überschuß) erhaltenes Diazomethin, versetzt mit 1 Gew.% des im Vergleichsbeispiel angegebenen Katalysators gepumpt und gleichzeitig je Mol Diazomethin 10 Mol Ammoniak zugeführt. Bei 140°C und einem durch kontinuierliche Zugabe von Wasserstoff bei 80 bar gehaltenen Gesamtdruck weist der den Reaktor am Kopf verlassende Reaktionsaustrag—wasser-, ammoniak- und katalysatorfrei gerechnet—folgende Produktzusammensetzung auf:

| | |
|---|---|
| n-Butylamin | 59,3% |
| 3(4)-Aminomethyltricyclo$[5.2.1.0^{2.6}]$-decan | 2,8% |
| 3(4),8(9)-Bis-(aminomethyl)tricyclo$[5.2.1.0^{2.6}]$-decan | 34,3% |
| 3(4)-Hydroxymethyl-8(9)-aminomethyltricyclo$[5.2.1.0^{2.6}]$-decan | 1,4% |
| Höhersieder | 2,2% |

Der Anfall an n-Butylamin deckt den Bedarf, der—auf die Zeiteinheit bezogen—für die Herstellung des Diazomethins notwendig ist.

Die Ausbeute an 3(4),8(9)-Bis-(aminomethyl)tricyclo[5.2.1.0$^{2.6}$]-decan beträgt rund 95% der Theorie, bezogen auf den eingebrachten Dialdehydanteil.

## Patentansprüche

1. Verfahren zur Herstellung von alliphatischen oder cycloaliphatischen Diaminen mit 4 bis 18 Kohlenstoffatomen, dadurch gekennzeichnet, daß man in einer ersten Stufe aliphatische oder cycloaliphatische Dialdehyde drucklos bei Temperaturen bis 100°C mit Monoaminen zu den entsprechenden Diazomethinen umsetzt und diese in einer zweiten Reaktionsstufe bei 60 bis 200°C und 50 bis 300 bar mit Ammoniak und Wasserstoff in Gegenwart von Hydrierungskatalysatoren in die Diamine überführt.

2. Verfahren nach Anspruch 1, dadurch gekennzeichnet, daß die Monoamine 3 bis 18 Kohlenstoffatome enthalten.

3. Verfahren nach Anspruch 1 und 2, dadurch gekennzeichnet, daß man das Monoamin in einem Überschuß von 10 bis 100 Mol%, bezogen auf den Dialdehyd, einsetzt.

4. Verfahren nach Anspruch 1 bis 3, dadurch gekennzeichnet, daß man den Dialdehyd anteilweise dem Amin zufügt.

5. Verfahren nach Anspruch 1 bis 4, dadurch gekennzeichnet, daß man als Hydrierungskatalysatoren Nickel- oder Kobaltkatalysatoren verwendet.

6. Verfahren nach Anspruch 1 bis 5, dadurch gekennzeichnet, daß je Mol Diazomethin 4 bis 20 Mole Ammoniak verwendet werden.

7. Verfahren nach Anspruch 1 bis 6, dadurch gekennzeichnet, daß die Umsetzung des Diazomethins bei 60 bis 160°C und 50 bis 250 bar erfolgt.

8. Verfahren nach Anspruch 1 bis 7, dadurch gekennzeichnet, daß die Diazomethinbildung und die anschließende Hydrierung in Gegenwart von Ammoniak kontinuierlich unter Rückführung des bei der Hydrierung gebildeten Monoamins durchgeführt wird.

## Revendications

1. Procédé de préparation de diamines aliphatiques ou cycloaliphatiques contenant de 4 à 18 atomes de carbone, caractérisé en ce que, dans un premier stade, on convertit des dialdéhydes aliphatiques ou cycloaliphatiques à pression normale, à des températures allant jusqu'à 100°C, en les diazométhines correspondantes par réaction avec des monoamines, et, dans un deuxième stade de réaction, on convertit les diazométhines en les diamines à une température de 60 à 200°C et une pression de 50 à 300 bars par réaction avec l'ammoniac et l'hydrogène en présence de catalyseurs d'hydrogénation.

2. Procédé selon la revendication 1, caractérisé en ce que les monoamines contiennent de 3 à 18 atomes de carbone.

3. Procédé selon les revendications 1 et 2, caractérisé en ce que la monoamine est mise en oeuvre en excès de 10 à 100 moles % par rapport au dialdéhyde.

4. Procédé selon les revendications 1 à 3, caractérisé en ce que l'on ajoute le dialdéhyde par portions à l'amine.

5. Procédé selon les revendications 1 à 4, caractérisé en ce que l'on utilise en tant que catalyseurs d'hydrogénation des catalyseurs au nickel ou au cobalt.

6. Procédé selon les revendications 1 à 5, caractérisé en ce que l'on utilise de 4 à 20 moles d'ammoniac par mole de diazométhine.

7. Procédé selon les revendications 1 à 6, caractérisé en ce que la diazométhine est convertie à une température de 60 à 160°C sous une pression de 50 à 250 bars.

8. Procédé selon les revendications 1 à 7, caractérisé en ce que la formation de la diazométhine et l'hydrogénation subséquente en présence d'ammoniac sont réalisées en continu avec recyclage de la monoamine formée à l'hydrogénation.

## Claims

1. Process for the production of aliphatic or cycloaliphatic diamines having 4 to 18 carbon atoms, characterized in that aliphatic or cycloaliphatic dialdehydes are reacted with monoamines in a first stage at atmospheric pressure and at temperatures up to 100°C to form the corresponding diazomethines and the latter are converted in a second reaction stage at 60 to 200°C and 50 to 300 bar with ammonia and hydrogen in the presence of hydrogenation catalysts into the diamines.

2. A process according to claim 1, characterized in that the monoamines contain 3 to 18 carbon atoms.

3. A process according to claims 1 and 2, characterized in that said monoamine is used in an excess of 10 to 100 mol. per cent, based on the dialdehyde.

4. A process according to claims 1 to 3, characterized in that the dialdehyde is added portionwise to the amine.

5. A process according to claims 1 to 4, characterized in that nickel or cobalt catalysts are used as hydrogenation catalysts.

6. A process according to claims 1 to 5, characterized in that 4 to 20 mols of ammonia are used per one mol of diazomethine.

7. A process according to claims 1 to 6, characterized in that the reaction of said diazomethine is carried out at 60° to 160°C and 50 to 250 bars.

8. A process according to claims 1 to 7, characterized in that the formation of diazomethine and the subsequent hydrogenation in the presence of ammonia are carried out continuously while recycling the monoamine produced in the hydrogenation.